(19) Europäisches Patentamt

European Patent Office

Office européen des brevets

(11) **EP 0 574 485 B1**

(12) **EUROPÄISCHE PATENTSCHRIFT**

(45) Veröffentlichungstag und Bekanntmachung des Hinweises auf die Patenterteilung:
**03.07.1996 Patentblatt 1996/27**

(21) Anmeldenummer: **92906401.2**

(22) Anmeldetag: **04.03.1992**

(51) Int. Cl.⁶: **A61B 5/04**, G01R 31/28

(86) Internationale Anmeldenummer:
**PCT/EP92/00475**

(87) Internationale Veröffentlichungsnummer:
**WO 92/15244 (17.09.1992 Gazette 1992/24)**

(54) **SCHALTUNGSANORDNUNG ZUR VERARBEITUNG VON PHYSIOLOGISCHEN MESSIGNALEN**

CIRCUIT ARRANGEMENT FOR PROCESSING PHYSIOLOGICAL MEASUREMENT SIGNALS

AGENCEMENT DE CIRCUITS DE TRAITEMENT DE SIGNAUX PHYSIOLOGIQUES DE MESURE

(84) Benannte Vertragsstaaten:
**CH DE FR GB IT LI NL SE**

(30) Priorität: **04.03.1991 DE 4106857**

(43) Veröffentlichungstag der Anmeldung:
**22.12.1993 Patentblatt 1993/51**

(73) Patentinhaber:
• **Siemens Elema AB**
**S-171 95 Solna 1 (SE)**

• **SIEMENS AKTIENGESELLSCHAFT**
**D-80333 München (DE)**

(72) Erfinder:
• **DANIELSSON, Peter**
**S-161 30 Bromma (SE)**
• **OHLSSON, Thomas**
**S-162 43 Vällingby (SE)**

(56) Entgegenhaltungen:
**DE-A- 2 429 955        US-A- 4 890 630**

## Beschreibung

Die Erfindung betrifft eine Schaltungsanordnung zur Verarbeitung von physiologischen Meßsignalen, welche mit Hilfe von Elektroden an einem Patienten abgegriffen werden, mit den Elektroden einzeln zugeordneten Eingangsverstärkern, die jeweils an einem ersten Eingangsanschluß mit den ihnen zugeordneten Elektroden und an einem zweiten Eingangsanschluß mit einem gemeinsamen Bezugspotentialanschluß verbunden sind, mit einer den Eingangsverstärkern, ausgangsseitig nachgeordneten Einrichtung zur Bildung von Differenzsignalen aus den Ausgangssignalen von jeweils zwei wählbaren Eingangsverstärkern und mit einem Kalibrierungsimpulsgenerator, der mit einer der Elektroden und dem Bezugspotentialanschluß verbunden ist und zwischen beiden einen Kalibrierungsimpuls erzeugt.

Bei einer derartigen, aus der DE-PS 24 29 955 bekannten Schaltungsanordnung erzeugt der dort vorhandenen Kalibrierungsimpulsgenerator einen Kalibrierungsimpuls definierter Höhe und Dauer zwischen den Eingangsanschlüssen der einzelnen Eingangsverstärker. In einer den Eingangsverstärkern ausgangsseitig nachgeordneten Einrichtung werden Differenzsignale aus den Ausgangssignalen von jeweils zwei wählbaren Eingangsverstärkern gebildet, so daR der Kalibrierungsimpuls aufgrund der Differenzbildung nicht in den betreffenden Differenzsignalen erscheint. Ist jedoch einer der Eingangsverstärker schadhaft, so erscheint in denjenigen Differenzsignalen, an deren Bildung das Ausgangssignal des schadhaften Eingangsverstärkers beteiligt ist, ein impulsförmiges Ausgangsfehlersignal. Bei der bekannten Schaltungsanordnung lassen sich zwar Fehler in den Eingangsverstärkern feststellen, nicht jedoch Fehler, die an den Elektroden und in deren Zuleitungen zu den Eingangsverstärkern auftreten können.

Der Erfindung liegt daher die Aufgabe zugrunde, die bekannte Schaltungsanordnung dahingehend zu verbessern, daß auch eine Überprüfung der Elektroden und ihrer Zuleitungen zu den Eingangsverstärkern möglich ist.

Gemäß der Erfindung wird die Aufgabe dadurch gelöst, daß bei der Schaltungsanordnung der eingangs angegebenen Art ein zusätzlicher Verstärker mit seinem Eingang an dem Bezugspotentialanschluß angeschlossen ist und daß der Ausgangsanschluß des zusätzlichen Verstärkers mit einer zusätzlichen Elektrode an dem Patienten verbunden ist. Bei der Abgabe eines Kalibrierungsimpulses durch den Kalibrierungsimpulsgenerator wird ein durch den Verstärkungsfaktor des zusätzlichen Verstärkers definierter Impulsanteil dem Patienten über die zusätzliche Elektrode zugeführt, während mit dem restlichen Impulsanteil der Bezugspotentialanschluß beaufschlagt wird. Da alle an dem Körper des Patienten angeordneten Elektroden über die Körperimpedanz elektrisch miteinander verbunden sind, erscheint der dem Patienten zugeführte Impulsanteil an allen diesen Elektroden. Bei der nachfolgenden Bildung von Differenzsignalen aus den Ausgangssignalen von jeweils zwei ausgewählten Eingangsverstärkern wird der betreffende Impulsanteil unterdrückt und tritt daher nicht in Erscheinung. Tritt jedoch in dem an der Bildung des Differenzsignales beteiligten Eingangsverstärker oder in der Elektrodenzuleitung zwischen dem Eingangsverstärker und der zugehörigen Elektrode ein Fehler auf, oder hat die betreffende Elektrode keinen richtigen Kontakt zu dem Körper des Patienten, so äußert sich dieser Fehler in Form eines Ausgangsimpulses in dem Differenzsignal.

Bei einer vorteilhaften Ausbildung der erfindungsgemäßen Schaltungsanordnung ist den Ausgängen der Eingangsverstärker eine Einrichtung zur digitalen Signalverarbeitung nachgeordnet, in der vor der eigentlichen Signalverarbeitung von den Ausgangssignalen der Eingangsverstärker der Kalibrierungsimpuls subtrahiert und den Signalen nach ihrer digitalen Verarbeitung wieder hinzuaddiert wird. Auf diese Weise wird der Kalibrierungsimpuls von der Signalverarbeitung ausgenommen, so daß insbesondere bei der Verwendung von Digitalfiltern diese nicht auf den Kalibrierungsimpuls ausgelegt werden brauchen, dessen Frequenzgehalt sich erheblich von dem der physiologischen Meßsignale unterscheidet.

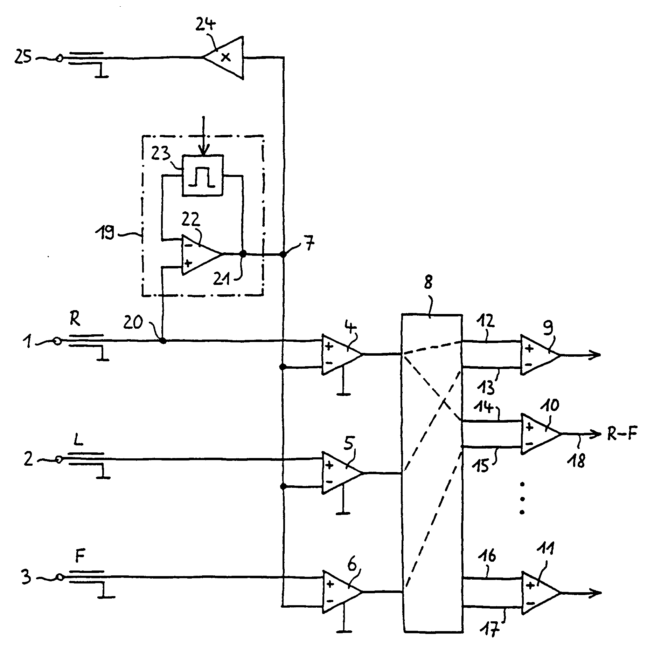

Zur Erläuterung der Erfindung wird im folgenden auf die Figur der Zeichnung Bezug genommen, die ein Elektrokardiografie-Gerät als Ausführungsbeispiel für die erfindungsgemäße Vorrichtung zeigt.

In der Figur sind drei Eingangskanäle R,L und F dargestellt, die zu drei am Körper des hier nicht gezeigten Patienten und zwar am rechten (R) und linken (L) Arm und an dem linken Fuß (F) angelegten Elektroden 1,2 und 3 führen. Zu diesen drei Eingangskanälen R,L und F kommen in bekannter Weise noch weitere Eingangskanäle für Elektroden an der Brustwand des Patienten, die jedoch der Übersichtlichkeit halber hier nicht dargestellt sind. Jeder der Elektroden 1,2 und 3 ist jeweils ein Eingangsverstärker 4,5 und 6 zugeordnet, der als Differenzverstärker mit einem ersten Eingangsanschluß (+) und einem zweiten Eingangsanschluß (-) ausgebildet ist. Dabei ist bei jedem der Eingangsverstärker 4,5 und 6 der jeweilige erste Eingangsanschluß (+) mit der zugeordneten Elektrode 1,2 bzw. 3 verbunden und die zweiten Eingangsanschlüsse (-) zu einem gemeinsamen Bezugspotentialanschluß 7 miteinander verbunden. Die Eingangsverstärker 4,5 und 6 sind ausgangsseitig gegebenenfalls über hier nicht gezeigte Filterstufen an einer Kanalwählereinheit 8 angeschlossen, der eine Mehrzahl von Differenzverstärkern 9,10 und 11 zugeordnet ist. Die Differenzverstärker 9,10 und 11 besitzen jeweils zwei Eingänge 12 und 13, 14 und 15 bzw. 16 und 17, wobei jedem Eingangspaar z.B. 14 und 15 die Ausgangssignale zweier von der Kanalwählereinheit 8 ausgewählter Eingangsverstärker z.B. 4 und 6 zuführbar sind. So wird bei dem dargestellten Beispiel an dem Ausgang 18 des Differenzverstärkers 10 das Differenzsignal R-F gebildet.

Zwischen den beiden Eingangsanschlüssen (+) und (-) des dem Kanal R bzw. der mit 1 bezeichneten Elektrode zugeordneten Eingangsverstärkers 4 ist ein Kalibrierungsimpulsgenerator 19 angeschlossen, der zwischen seinen beiden Anschlüssen 20 und 21 auf Anforderung einen Spannungsimpuls definierter Höhe (vorzugsweise 1mV) und Dauer erzeugt. Um den Kanal R elektrisch nicht zu belasten, ist der Kalibrierungsimpulsgenerator 19 als Impedanzwandler 22 mit einem Spannungsimpulsgeber 23 im Rückkopplungszweig ausgebildet. An dem allen Eingangsverstärkern 4,5 und 6 gemeinsamen Bezugspotentialanschluß 7 liegt also ein Potential an, das dem Potential an der Elektrode 1 überlagert mit dem Spannungsimpuls des Kalibrierungsimpulsgenerators 19 entspricht. Dieses Potential ist daher mit denselben Störsignalen überlagert, die von den Elektroden 1,2 und 3 erfaßt werden; da die Eingangsverstärker 4,5 und 6 Differenzverstärker sind, werden diese Störsignale nicht verstärkt sondern unterdrückt. Wird ein Kalibrierungsimpuls erzeugt, so ändert sich an den Anschluß 7 das Bezugspotential für alle Eingangsverstärker 4,5 und 6 in gleicher Weise. Da durch die Differenzverstärker 9,10 und 11 die Differenz zwischen je zwei Ausgangssignalen der Eingangsverstärker 4,5 und 6 gebildet wird, erfolgt durch die Veränderung des Bezugspotentials an dem Anschluß 7 keine Veränderung der Ausgangssignale der Differenzverstärker 9 bis 11, so lange die Eingangsverstärker 4,5 und 6 in Ordnung sind. Ist dagegen einer der Eingangsverstärker 4,5 und 6 schadhaft, so erscheint an demjenigen der Differenzverstärker 9,10 und 11 ein Ausgangsfehlersignal, der von dem schadhaften Eingangsverstärker ein Eingangssignal erhält.

Wird beispielsweise die von dem Kalibrierungsimpulsgenerator 19 erzeugte Spannung zwischen den Anschlüssen 20 und 21 mit U bezeichnet und die von den Elektroden 1,2 und 3 abgenommenen Potentiale mit R,L und F, dann hat das Potential an dem Bezugspotenzialanschluß 7 den Wert R-U und zwischen den Eingangsanschlüssen (+) und (-) der Eingangsverstärker 4,5 und 6 liegen die Eingangsspannungen U, L-R+U und F-R+U . Dabei ergibt sich zwischen den Ausgangssignalen der Eingangsverstärker 4 und 5 das Differenzsignal L-R, bei den Eingangsverstärkern 5 und 6 das Differenzsignal L-F und bei den Eingangsverstärkern 4 und 6 das Differenzsignal F-R, vorausgesetzt, daß die Eingangsverstärker 4,5 und 6 in Ordnung sind.

Die dargestellte Schaltungsanordnung entspricht - soweit sie bis hierhin beschrieben wurde - der aus der DE-PS 24 29 955 bekannten Anordnung. Wie die bisherige Beschreibung zeigt, lassen sich dabei zwar Fehler in den Eingangsverstärkern 4,5 und 6 feststellen, nicht jedoch an den Elektroden 1,2 und 3 und deren Zuleitungen bis zu den Eingangsverstärkern 4,5 und 6. Um dies zu erreichen ist ein zusätzlicher Verstärker 24 mit seinem Eingang an dem Bezugspotentialanschluß 7 angeschlossen und an seinem Ausgang mit einer zusätzlichen Elektrode 25 verbunden, die am Körper des Patienten, z.B. an seinem rechten Fuß angeordnet ist. Das an dem Bezugspotentialanschluß 7 anliegende Potential wird auf diese Weise verstärkt und dem Körper des Patienten über die Elektrode 25 zugeführt. Wird dabei der von dem Kalibrierungsimpulsgenerator erzeugte Spannungsimpuls mit U bezeichnet und der Verstärkungsfaktor des Verstärkers 24 mit x angegeben, so wird der Patient mit einem Impulsanteil der Höhe U.x/(x- 1) beaufschlagt, während der an dem Bezugspotentialanschluß 7 auftretende Impulsanteil die Höhe U/(x-1) aufweist. Da die Elektroden 1,2,3 und 25 über die Körperimpedanz des Patienten elektrisch leitend miteinander verbunden sind, erscheint der Impulsanteil U.x/(x-1) an allen Elektroden 1,2,3 und 25. Da sich ferner beim Auftreten des Kalibrierungsimpulses die Potentialverhältnisse an den Elektroden 1,2 und 3 in gleicher Richtung und im gleichen Umfang wie an dem Bezugspotentialanschluß 7 ändern, erscheinen wie oben bereits gezeigt an den Eingangsverstärkern 4,5 und 6 die Eingangsspannungen U, L-R+U und F-R+U , so daß die Differenzsignale zwischen den Ausgängen der Eingangsverstärker 4 und 5, 5 und 6, sowie 4 und 6 unverändert L-R, L-F bzw. F-R betragen. Tritt jedoch in einem der Eingangsverstärker 4,5 oder 6 oder in einer der Elektrodenzuleitungen zwischen den Eingangsverstärkern 4,5 und 6 und den Elektroden 1,2 und 3 ein Fehler auf, oder hat eine der Elektroden 1,2 oder 3 keinen richtigen Kontakt zum Patientenkörper, so äußert sich dieser Fehler in Form eines Ausgangimpulses an demjenigen der Differenzverstärker 9,10 und 11, der über die Kanalwählereinheit 8 mit dem betreffenden fehlerhaften Kanal R,L oder F verbunden ist.

Bei dem dargestellten Ausführungsbeispiel erfolgt die Bildung von Diffenzsignalen aus den Ausgangssignalen der Eingangsverstärker 4,5 und 6 auf analogem Wege. Auf welche Weise dabei auch die Funktionsfähigkeit der Differenzverstärker 9,10 und 11 überprüft werden kann, geht aus der bereits genannten DE-PS 24 29 955 hervor. Alternativ zur analogen Signalverarbeitung können die Ausgangssignale der Eingangsverstärker 4,5 und 6 auch auf digitalem Wege weiter verarbeitet werden. Da wie oben bereits beschrieben der Kalibrierungsimpuls erst bei der Bildung der Differenzsignale aus den Ausgangssignalen der Eingangsverstärker verschwindet, müßte bei der digitalen Signalverarbeitung der Kalibrierungsimpuls bei allen Verfahrungsschritten, die einer Differenzbildung vorausgehen mitverarbeitet werden. Insbesondere bei einer digitalen Signalfilterung wäre dies störend, da der Kalibrierungsimpuls ein anderes Frequenzspektrum als die physiologischen Signale aufweist. Außerdem müßte bei einer komplexen Signalverarbeitung unterschieden werden, ob der Kalibrierungsimpuls aufgrund von Differenzbildungen verschwindet oder anderenfalls erhalten bleibt. Daher wird bei der digitalen Signalverarbeitung zunächst aus den Ausgangssignalen der Eingangsverstärker 4,5 und 6 der Kalibrierungsimpuls heraussubtrahiert; danach erfolgt die digitale Signalverarbeitung an deren Ende ein neuer Kalibrierungsimpuls hinzuaddiert wird. Auf diese Weise wird der Kalibrierungsimpuls von der digitalen Signalverarbeitung ausgenommen. Nur wenn im Eingangsverstärkerkreis ein Fehler vorliegt, verbleibt nach der Subtraktion ein Restimpuls, der in die Signalverarbeitung eingeht.

**Patentansprüche**

1. Schaltungsanordnung zur Verarbeitung von physiologischen Meßsignalen, welche mit Hilfe von Elektroden (1,2,3) an einem Patienten abgegriffen werden, mit den Elektroden (1,2,3) einzeln zugeordneten Eingangsverstärkern (4,5,6), die jeweils an einem ersten Eingangsanschluß (+) mit den ihnen zugeordneten Elektroden (1,2,3) und an einem zweiten Eingangsanschluß (-) mit einem gemeinsamen Bezugspotentialanschluß (7) verbunden sind, mit einer den Eingangsverstärkern (4,5,6) ausgangsseitig nachgeordneten Einrichtung (8,9,10,11) zur Bildung von Differenzsignalen aus den Ausgangssignalen von jeweils zwei wählbaren Eingangsverstärkern und mit einem Kalibrierungsimpulsgenerator (19), der mit einer der Elektroden (z.B.1) und dem Bezugspotentialanschluß (7) verbunden ist und zwischen beiden einen Kalibrierungsimpuls erzeugt, **dadurch gekennzeichnet**, daß ein zusätzlicher Verstärker (24) mit seinem Eingang an dem Bezugspotentialanschluß (7) angeschlossen ist und daß der Ausgangsanschluß des zusätzlichen Verstärkers (24) mit einer zusätzlichen Elektrode (25) an dem Patienten verbunden ist.

2. Schaltungsanordnung nach Anspruch 1, **gekennzeichnet** durch eine den Ausgängen der Eingangsverstärker (4,5,6) nachgeordnete Einrichtung zur digitalen Signalverarbeitung, in der vor der Signalverarbeitung von den Ausgangssignalen der Eingangsverstärker (4,5,6) der Kalibrierungsimpuls subtrahiert und den Signalen nach ihrer digitalen Verarbeitung wieder hinzuaddiert wird.

**Claims**

1. Circuit arrangement for the processing of physiological measurement signals, which are picked off by means of electrodes (1, 2, 3) on a patient, having input amplifiers (4, 5, 6) which are individually associated with the electrodes (1, 2, 3) and which are connected in each instance at a first input connection (+) to the electrodes (1, 2, 3) associated with them and at a second input connection (-) to a common reference potential connection (7), having a device (8, 9, 10, 11), disposed downstream of the input amplifiers (4, 5, 6) on the output side, for the formation of difference signals from the output signals of in each instance two selectable input amplifiers and having a calibration pulse generator (19) which is connected to one of the electrodes (e.g. 1) and the reference potential connection (7) and generates between the two a calibration pulse, characterized in that an additional amplifier (24) is connected by its input to the reference potential connection (7), and in that the output connection of the additional amplifier (24) is connected to an additional electrode (25) on the patient.

2. Circuit arrangement according to Claim 1, characterized by a device, disposed downstream of the outputs of the input amplifiers (4, 5, 6), for digital signal processing, in which prior to the signal processing the calibration pulse is subtracted from the output signals of the input amplifiers (4, 5, 6) and is added again to the signals after their digital processing.

**Revendications**

1. Montage de traitement de signaux physiologiques de mesure, qui sont prélevés d'un patient à l'aide d'électrodes (1,2,3), comportant des amplificateurs (4,5,6) d'entrée qui sont associés individuellement aux électrodes (1,2,3) et qui sont reliés en une première borne (+) d'entrée respectivement aux électrodes (1,2,3) qui leur sont associées et en une seconde borne (-) d'entrée à une borne (7) commune de potentiel de référence, comportant un dispositif (8,9,10,11) qui est disposé en aval du côté sortie des amplificateurs (4,5,6) d'entrée et qui est destiné à la formation de signaux de différence à partir des signaux de sortie de respectivement deux amplificateurs d'entrée pouvant être choisis et comportant un générateur (19) d'impulsions d'étalonnage, qui est relié à l'une des électrodes (par exemple 1) et à la borne (7) de potentiel de référence et qui forme entre les deux une impulsion d'étalonnage, caractérisé en ce qu'un amplificateur (24) supplémentaire est connecté par son entrée à la borne (7) de potentiel de référence et la borne de sortie de l'amplificateur supplémentaire (24) est reliée à une électrode supplémentaire (25) sur le patient.

2. Montage suivant la revendication 1, caractérisé par un dispositif de traitement numérique de signaux, qui est disposé en aval des sorties des amplificateurs (4,5,6) d'entrée et dans lequel l'impulsion d'étalonnage est soustraite, avant le traitement des signaux, des signaux de sortie des amplificateurs (4,5,6) d'entrée et est réadditionnée aux signaux après leur traitement numérique.